# EUROPEAN PATENT APPLICATION

(11) **EP 1 541 590 A1**
(43) Date of publication of application: **15.06.2005**
(21) Application number: 03028155.4
(22) Date of filing: 08.12.2003
(51) Int. Cl.: C08B 15/02, C08B 15/04, C08B 37/00, C08B 37/18, C08B 31/18, C08B 33/08, C08B 35/08, C07B 41/08, C07C 45/37, C07C 51/23, C07H 7/033, C07D 307/50, D21C 9/00

(54) **Process for the oxidation of hydroxy compounds by means of nitroxy compounds**

(71) Applicant: SCA Hygiene Products AB, 405 03 Göteborg (SE)
(72) Inventor: Jetten, Jan, 3701 JL Zeist (NL); Besemer, Arie, 3958 CC Amerongen (NL)
(74) Representative: HOFFMANN EITLE

(57) **Abstract**

The present invention relates to a process for the oxidation of a hydroxy compound comprising the steps of:
a) providing a first mixture containing an organic protonated hydroxylamine compound,
b) oxidizing said protonated hydroxylamine compound to the corresponding nitrosonium compound,
c) reacting the nitrosonium compound obtained thereby with a second mixture containing a hydroxy compound, and
d) oxidizing said hydroxy compound by means of the nitrosonium compound, thereby generating the hydroxylamine.

## Description

The present invention relates to a process for the oxidation of hydroxy compounds by means of nitroxy compounds.

### Background art

Nitroxy-mediated oxidations are a useful tool to convert primary hydroxy compounds to aldehydes and/or carboxylic acids or secondary hydroxy compounds to ketones. Especially the oxidation by means of TEMPO (2,2,6,6-tetramethylpiperidine-N-oxyl, formula I in the scheme below) has attracted a lot of interest over the last years since it allows the selective introduction of aldehyde and/or carboxylic acids into hydroxy compound substrates such as polysaccharides.

The reaction can be conducted in three ways. First, a nitrosonium ion (III), which is the actual active species, can be generated catalytically in situ from a nitroxide in the presence of a primary oxidant, such as hypochlorous acid/hypochlorite as shown in the scheme below. Depending on the process conditions, the nitrosonium ion will oxidize the alcohol, i.e. the hydroxy compound to the corresponding aldehyde (as shown in the scheme) or carboxy compound while being reduced itself to the hydroxylamine. Equimolar amounts of the resulting hydroxylamine and nitrosonium ion will then recombine (synproportionate) under suitable reaction conditions to form the nitroxy compound. Then, the catalytic cycle newly starts.

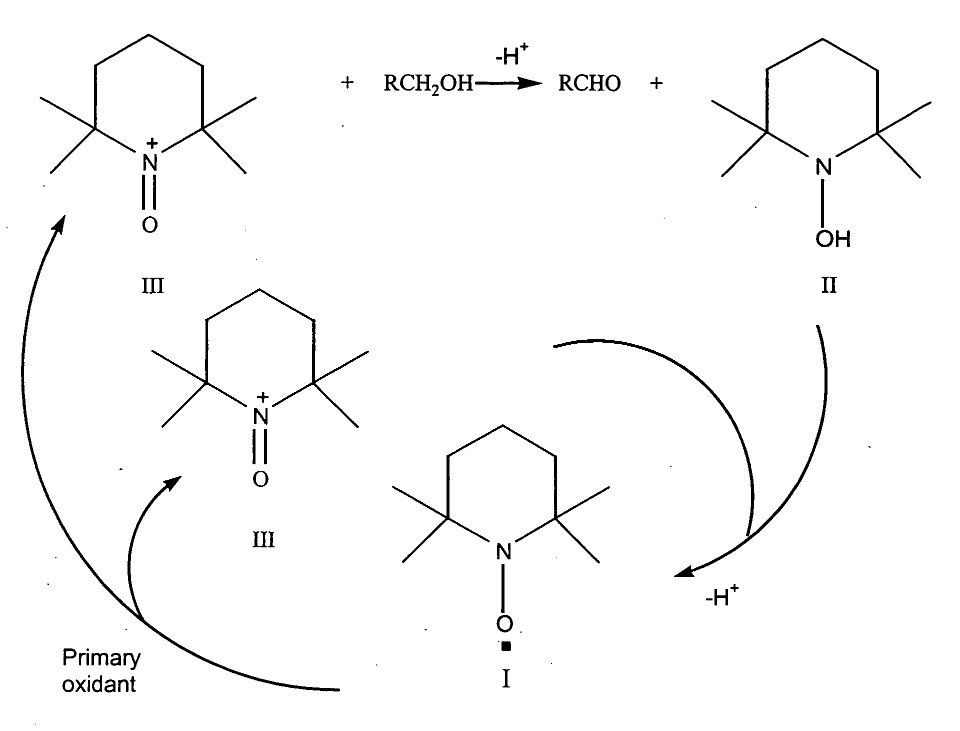

Second, the nitrosonium ion can be generated in situ by a disproportionation reaction of the nitroxy compound (I) to the corresponding hydroxylamine (II) and the nitrosonium ion (III) as shown in the following scheme. As the disproportionation reaction must be conducted under (strongly) acidic conditions, the hydroxylamine (II) is typically present in its protonated form (IV).

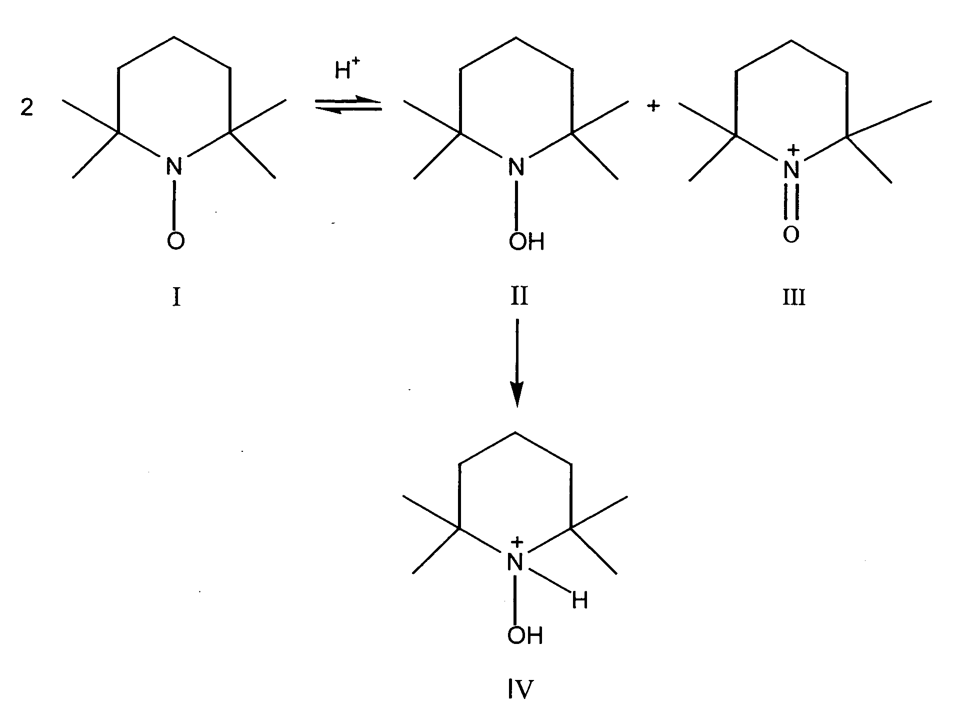

Third, it is also possible to prepare a salt of the nitrosonium ion, which then can be used in stoichiometric amounts.

Moreover, a relatively new technique involves the enzymatic conversion of nitroxy compounds as described in WO 00/50463.

There are a very high number of patent and scientific publications dealing with the synthesis and the use of nitroxy compounds, mostly TEMPO and its derivatives in oxidation reactions.

Reviews were published for instance by J.M. Bobbitt et al. in Heterocycles, vol. 27, No. 2, 1988 "Organic nitrosonium salts as oxidants in organic chemistry" and by A.E.J. De Nooy et al. in J. Synthetic Org. Chem 1996 (10),1153-1174 "On the use of stable organic nitroxyl radicals for the oxidation of primary and secondary hydroxy compounds".

If TEMPO and certain related nitroxy compounds are used as a catalyst in the oxidation of alcohols, for instance oligo- or polysaccharides, in aqueous suspensions and solutions, the nitroxy compound is often partially lost during the isolation of the reaction product due to the relatively high volatility of some nitroxy compounds such as TEMPO. This is an undesired situation for an economical process. Very few documents deal with technical solutions for overcoming this problem or the recovery of TEMPO in general.

WO 96/36621 proposes in this regard a method for obtaining a catalytically active mixture based on stable nitroxide radicals wherein at least a part of the reaction mixture is subjected to an azeotropic distillation. During the azeotropic distillation step volatile nitroxide radicals are at least partially distilled over with the liquid medium. However, this method requires distillation devices and creates high costs due to its energy consumption. Further it is not applicable to less volatile nitroxy derivatives.

Another technique for avoiding the loss of TEMPO as a catalyst involves affixing the same to a polymer solid phase which then can be easily recovered from the reaction medium (T. Miyazawa et al. in J. Polymer Sci. Polymer Chem. Ed. 23 (1985) 1527-1535 and 2487-2494). Similarly, DE 4209869 A describes a method for immobilizing 4-hydroxy-TEMPO on polyvinyl-benzyl.

US 2003/0073871 pertains to the continuous oxidation of hydroxy compound substrates to aldehydes in the presence of nitroxy compounds in an alkaline 2-phase system. The nitroxy compound can be present in the organic phase, in the aqueous phase or bound to solid supports.

However, the reaction rate of immobilized organic nitroxy compounds is typically lower than in homogeneous systems.

WO 02/59064 A1 relates to various recovery processes for TEMPO and related nitroxy compounds based on hydrophobic interactions. One option involves bringing into contact the nitroxy compound with hydrophobic resins such as XAD resins, for instance by passing the reaction mixture over a chromatographic column filled with one of these resins. The hydrophobic character of TEMPO and other organic nitroxy compounds leads to a strong affinity to hydrophobic resins allowing the separation from the reaction mixture. Nevertheless, this recovery process creates new problems insofar as, after extracting the organic nitroxy compound from the column, the organic solvent (eluent) has to be removed by evaporation. At this point in time, the high volatility of some organic nitroxy compounds such as TEMPO or 3,4-dehydro-TEMPO leads again to an undesired loss of this costly material.

WO 02/58844 describes a special group of nitroxy compounds wherein 4 TEMPO molecules are integrated into a heterocyclic molecule and their use in the oxidation of hydroxy compound substrates. These nitroxy compounds can be recovered by filtration or water evaporation steps. One method for the recovery of the nitroxy compound catalyst involves the filtration at pH values above 8. According to another embodiment, the organic phase containing the catalyst is stirred with concentrated hydrochloric acid (HCl) to transfer the catalyst into the aqueous phase followed by evaporating the water under formation of the catalyst salt.

Further, it is known in the art that N-methylmorpholine-N-oxide (NMMO) which is used as solvent in the Lyocell spinning process can be recovered and purified by means of anion exchange resins (H. Mertel et al., Papier (Darmstadt), 46 (3), 101-5, 1992, "Purification, recovery and methods for quantitative determination of N-methylmorpholine-N-oxide"). The separation of tertiary amine oxides, such as NMMO from aqueous solutions by means of cationic exchange resins having carboxylate anchor groups is further known from EP 0 468 951 A1.

In view of the above, there is a strong need for a nitroxy-mediated oxidation process that can be conducted in a closed cycle, but avoids the typical problems associated with the recovery of TEMPO and related organic nitroxy compounds.

Further some hydroxy compound substrates are relatively sensitive towards the unspecific oxidation by the primary oxidant which leads to undesired side products.

Therefore, it is one object of the present invention to provide an efficient oxidation process mediated by TEMPO and related organic nitroxy compounds that can be conducted in a closed cycle.

According to a further aspect of the present invention, a process for the oxidation of hydroxy compounds is to be provided wherein organic nitroxy compounds can be used without substantial material losses.

It is a further object of the present invention to provide a nitroxy-mediated oxidation process for hydroxy compounds wherein the active species (the nitrosonium ion) can be generated separately from the reaction mixture containing the hydroxy compound to be oxidized in order to minimize undesired side reactions.

### Brief description of the invention

The above technical objects are solved by
a process for the oxidation of a hydroxy compound comprising the steps of:
a) providing a first mixture containing an organic protonated hydroxylamine compound,
b) oxidizing said protonated hydroxylamine compound to the corresponding nitrosonium compound,
c) reacting the nitrosonium compound obtained thereby with a second mixture containing a hydroxy compound, and
d) oxidizing said hydroxy compound by means of the nitrosonium compound, thereby generating the hydroxylamine.

### Detailed description of the invention

According to the present invention, there are no specific limitations regarding the hydroxy compound to be oxidized. It is one feature of nitroxy-mediated oxidations that they are compatible with a huge variety of different hydroxy compound substrates. Thus, the hydroxy functionality can be either primary or secondary. The oxidation of primary hydroxy compounds is preferred since techniques known in the art allow conducting the nitroxy-mediated oxidation of primary hydroxy compounds with particularly high selectivities and conversion rates. The primary hydroxy compound is oxidized to the corresponding aldehyde and/or carboxylic acid. The interruption of the oxidation process and/or a suitable reaction conditions enable the isolation of the intermediate aldehyde. Intrruption of the oxidation process is particularly suited for the oxidation of polysaccharides.

Examples for suitable hydroxy compound substrates are low molecular weight (up to MW 1000) aliphatic or aromatic-aliphatic hydroxy compounds, such as oligosaccharides, as well as hydroxy compounds having a higher molecular weight including polymeric hydroxy compounds such as oligo- and polysaccharides. The oxidation of oligo- and polysaccharides, such as starch or cellulose typically leads to the corresponding C6-aldehyde and/or carboxy derivates. The oxidation of cellulose and cellulose- containing materials, such as pulp is particularly preferred.

The starting pulps which may be used for oxidation may relate to primary fibrous materials (raw pulps) or to secondary fibrous materials, whereby a secondary fibrous material is defined as a fibrous raw material recovered from a recycling process. The primary fibrous materials may relate both to a chemically digested pulp (e.g. Kraft or sulfite pulp) and to mechanical pulp such as thermorefiner mechanical pulp (TMP), chemothermorefiner mechanical pulp (CTMP) or high temperature chemithermomechanical pulp (HTCTMP). Synthetic cellulose-containing fibers can also be used. Preference is nevertheless given to the use of pulp from plant material, particularly wood-forming plants. Fibers of softwood (usually originating from conifers), hardwood (usually originating from deciduous trees) or from cotton linters can be used for example. Fibers from esparto (alfa) grass, bagasse (cereal straw, rice straw, bamboo, hemp), kemp fibers, flax and other woody and cellulosic fiber sources can also be used as raw materials. The corresponding fiber source is chosen in accordance with the desired properties of the end product in a manner known in the art.

The oxidized pulps can be used for the manufacture of paper, in particular tissue paper having improved strength properties. Prior to or after oxidation the starting pulps can be beaten (refined) with the aim of further enhancing paper strength. In the manufacture of oxidized pulps it is further preferred to carry out all oxidation steps in the absence of chlorine-containing oxidants as basis for the production of TCF or ECF paper.

According to **step (a)** of the claimed process a mixture containing a protonated hydroxylamine is provided.

This mixture can be an aqueous or organic dispersion or preferably an aqueous or organic solution. Suitable organic solvents can be selected from water-miscible organic solvents such as alcohols (e.g. ethanol, 1- or 2- propanol, t-butanol), ketones (e.g. acetone), ethers (e.g. THF, dioxane), or nitriles (e.g. CH₃CN) (the term "water-miscible" refers to a complete mutual solubility at 20°C).

The content of organic hydroxylamine compound in the solution is only limited by the solubility. Depending on the type of organic hydroxylamine compound to be dissolved, this amount preferably ranges from 0.1 to 30 wt%, preferably 1 to 10 wt%, based on the weight of the solvent (mixture).

The protonated hydroxylamine is preferably produced from the corresponding nitroxy compound, either by reducing or preferably by disproportionating the same.

The disproportionation reaction of nitroxy compounds is achieved by adjusting the pH value to a value below 4, preferably below 3, more preferably below 2 with a suitable acid (throughout the specification and claims pH values refer to a measurement at 20°C). At these pH values nitroxy compounds undergo a disproportionation reaction leading to the corresponding oxdized species (nitrosonium ion) and the reduced species (hydroxylamine) the latter being automatically protonated. In contrast to the nitroxy compound itself, the generated charged species, i.e. the nitrosonium ion and the protonated hydroxylamine are not volatile. Therefore, in the following steps, solvents can be removed, if necessary, without any substantial loss of material.

For adjusting the pH to acidic values causing disproportionation any acid can be used, e.g. perchloric acid, although it is generally preferred to employ non-oxidising and non-reducing organic or inorganic acids. In order to reach the necessary low pH values, it is generally advantageous to use stronger acids, for instance those having pK values (at 25°C in an aqueous solution) of less than 5, preferably less than 3, for instance less than 1 in particular less than -1. Very strong acids fulfilling even the latter requirement are sulfuric acid or hydrochloric acid. If the entire oxidation process is to be conducted under TCF (total chlorine free) conditions, it is preferred to use chlorine-free acids, such as organic sulfonic acids (e.g. benzenesulfonic acid, o- or p- toluenesulfonic acid), sulfuric acid, nitric acid or phosphoric acid.

The type of acid used in this disproportionation step will also determine the counter-ion of the hydroxylamine compound.

According to step (a), it is not only possible to use fresh nitroxy compounds for generating the protonated hydroxylamine. Alternatively, and preferably, unreacted nitroxy compound stemming from a reaction mixture, wherein an hydroxy compound has been oxidized in the presence of said nitroxy compound, can be used.

The hydroxylamine compound is preferably derived from a sterically hindered organic nitroxy compound. Furthermore, the use of secondary nitroxides is preferred (in contrast thereto, NMMO, i.e. N-methyl morpholin-N-oxide is for instance a tertiary nitroxide). (This, as well as the following description of structural features, also naturally applies to the corresponding nitrosonium and hydroxylamine compounds). One, particularly two bulky groups in the α position to the NO is/are suitable for sterical hindrance of the NO group, e.g. optionally substituted phenyl or aliphatic substituents that are linked to the nitrogen atom of the NO by a quaternary C atom, e.g. tert-butyl. In other words, it is preferred that one, in particular two quaternary carbon atoms are present in α-position of the N-atom. According to preferred embodiments, one, in particular both α-carbon atoms are dimethyl-substituted. It is similarly preferred that the nitroxy compound lacks α-hydrogen atoms. Two substituents can also be combined into an alkylen unit optionally interrupted by a hetero-atom (e.g. O,N) (to form an alicyclic or heterocyclic ring). The molecular weight of the nitroxy compound is preferably less than 500, its carbon number preferably less than 40, in particular less than 30, e.g. less than 20.

Preferred nitroxy precursor systems for the hydroxylamine compound can be represented by the following formula (V) 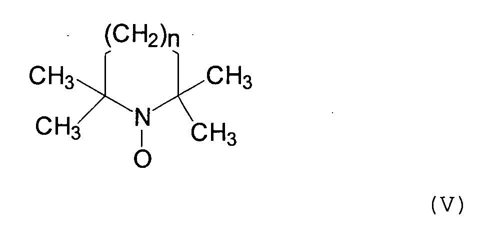 where n = 0 or 1 and where the methylene groups of the ring may carry one or more substituents selected from alkyl, alkoxy, aryl, aryloxy, amino, amido (e.g. acetamido, 2-bromacetamido and 2-iodacetamido), oxo, cyano, hydroxy, carboxyl, phosphonooxy, maleimido, isothiocyanato, alkyloxy, fluorophosphinyloxy (particularly ethoxyfluorophosphinyloxy), substituted or unsubstituted benzoyloxy, e.g. 4-nitrobenzoyloxy. If n = 1 (i.e. the ring represents a piperidine), these groups typically substitute the 4-position of the piperidine. Examples are 4-acetamido-TEMPO, 4-acetoxy-TEMPO or 4-hydroxy-TEMPO. The di-tert.-alkyl nitroxy unit can also be present as part of a polymer structure such as {(CH₃)₂-C-(CH₂)₂₋₃-(CH₃)₂-C-NO-}ₘ-. Hydroxy, amino and amido are preferred among these substituents on account of the stability of the nitroxy compound under acidic conditions. The ring may also be partially unsaturated as in 3,4-dehydro-TEMPO.

An example of n=0 is PROXYL (2,2,5,5-tetramethylpyrrolidine-N-oxyl).

DOXYL (4,4-dimethyloxazolidine-N-oxyl) can also be used as nitroxy compound in the present invention.

Examples for volatile organic nitroxy compound, for which the claimed method is particularly suitable, comprise TEMPO itself and 3,4-dehydro-TEMPO, but are not limited thereto.

According to **step (b)** of the claimed process, the protonated hydroxylamine is oxidized to the corresponding nitrosonium ion at a pH of 7 or below, more preferably 6 or below , most preferably 5 or below. It should be added that, from a strictly mechanistical point of view, then non-protonated hydroxylamine per se may represent the actual oxidized species. However, due to the existing equilibrium between protonated "free" hydroxylamine, the net result is the same.

This step aims at converting the protonated hydroxylamine to the corresponding nitrosonium ion as far as possible, preferably to an extent of at least 80 mol%, more preferably of at least 90 mol%, e.g. at least 95 mol%.

For this purpose, an essentially stoichiometric amount of a suitable oxidant may be added to the protonated hydroxylamine. "Essentially stoichiometric" means a stoichiometric amount ± 20%, preferably ± 10% of the stoichiometrically required molar amount. This ensures that only small amounts of the oxidant remain in the reaction mixture obtained in step (b).

Alternatively, the oxidant is added in a molar excess with respect to the stoichiometrically required amount, for instance in amounts of more than 1.2 mol, more preferably more than 1.5 mol, even more preferably more than 2 mol (e.g. up to 5 mol), if the stoichiometrically required amount is assumed to be 1. Then, it is preferred to reduce this excess prior to step (c).

Suitable oxidants can be selected among primary oxidants typically used together with organic nitroxy compounds such as chlorine, bromine, iodine, hypochlorite, chlorite (in combination with hypochlorite), hypobromite, Fe(CN)₆³⁻, transition metals of periods Va to VIIIa in the oxidation state of at least +3, oxidases, ozone, hydrogen peroxide, peroxosulfate and/or peracids, hypochlorite and peracid being preferred. For each of these primary oxidants suitable reaction conditions, as known in the art, are to be selected. Thus, it can for instance be undesired to use hypochlorite at pH <2 since then chlorine is formed, and tends to escape from the reaction mixture.

A further version lies in the oxidation of the hydroxylamine with a peracid, a precursor or a salt thereof as a primary oxidizing agent, in the presence of a catalytic amount of a halide (e.g. NaBr), preferably in the pH range of 2 to 7, particularly 2.5 to 3.5, similarly as disclosed in WO 99/57158. The peracid is preferably a peralkanoic acid, particularly peracetic acid, or persulfuric acid. It is, however, also possible to perform oxidation just using peracid (e.g. persulfuric acid) as an oxidizing agent without halide.

Another version lies in the reaction between hydroxylamine and a suitable oxidic compound of a metal of the transition metals of periods Va to VIIIa in the oxidation state of at least +3, e.g. oxides and oxygen-containing ions of manganese, chromium, iron, nickel, ruthenium and vanadium, e.g. vanadium pentoxide, iron oxide, chromium (VI) oxide, chromates and particularly manganese (IV) oxide and salts of permanganic acid. The reaction is preferably conducted at a pH between 2 and 7. The reaction temperature is preferably less than 80°C, particularly 30 to 60°C. This technique is similar to the description of WO 01/00681.
Another preferred version lies in the oxidation of hydroxylamine compounds, preferably those derived from 4-hydroxy-, 4-amino- or 4-amido-substituted nitroxy TEMPO) at a pH between 1 and 4, particularly 2 to 4. In this version, a hypohalite (e.g. NaOCl) or ozone is particularly suitable as a primary oxidizing agent. The reaction temperature is preferably 5 to 50°C. Halogen-free acids, such as sulfuric acid or toluenesulfonic acid, are particularly suitable for setting the pH, although hydrochloric acid may also be used.

Finally, the hydroxylamine can also be oxidized with enzymes and/or metal complexes. The enzymes to be used according to this embodiment are oxidoreductases or other enzymes that are capable of oxidation in the presence of a suitable redox system. Oxido-reductases, i.e. enzymes capable of oxidation without the presence of further redox systems, to be used include peroxidases and oxidases, in particular polyphenol oxidases and laccase. Certain hydrolases, such as phytase, can be used when a further redox system is present such as a metal complex, e.g. vanadate. Metal complexes as such, without an enzyme protein, can also be used; examples include copper and iron complexes with porphyrins, phenanthrolins, polyamines such as EDTA, EGTA and the like. The metal-assisted enzymes and metal complexes require hydrogen peroxide, alkyl and ar(alk)yl hydroperoxides (such as tert-butyl hydroperoxide) or chlorite as an ultimate electron acceptor. Peroxidases (EC 1.11.1.1 - 1.11.1.11) that can be used according to the invention include the peroxidases which are cofactor-independent, in particular the classical peroxidases (EC 1.11.1.7). Peroxidases can be derived from any source, including plants, bacteria, filamentous and other fungi and yeasts. Examples are horseradish peroxidase, soy-hull peroxidase, myelo peroxidase, lactoperoxidase, Arthromyces and Coprinus peroxidases. Several peroxidases are commercially available. The peroxidases require hydrogen peroxide as an electron acceptor. Polyphenol oxidases (EC 1.10.3.1.) include tyrosinases and catechol oxidases such as lignine peroxidase. Suitable polyphenol oxidases may be obtained from fungi, plants or animals. The polyphenol oxidases require oxygen as an electron acceptor. Laccases (EC 1.10.3.2) are sometimes grouped under the polyphenol oxidases, but they can also be classified as a distinct group, sometimes referred to as p-diphenol oxidases. The laccases can be derived from plant sources or from microbial, especially fungal, sources. The laccases also require oxygen as an electron acceptor. The process for producing the nitrosonium ion according to this embodiment can be performed under relatively mild conditions, e.g. at a pH between 2 and 7, and at a temperature between 15 and 60°C (both depending on the particular enzyme or metal complex). The reaction medium can be an aqueous medium.

The use of hypochlorous acid is most preferred. If hypochlorous acid has been used in excess of the stoichiometrically required amount, it can be decomposed with hydrogen peroxide (here as reducing agent). This has the decisive advantage that the mixture to be reacted with the hydroxy compound substrate, e.g. a cellulosic pulp does not contain hypochlorite/chlorine but only the nitrosonium ion and sodium chloride. Thus, a pulp oxidation conducted in this manner can be considered as a chlorine-free process.

Step (b) of the claimed process involves the direct oxidation of hydroxylamine to the corresponding nitrosonium ion. Thus, the formation of the intermediate nitroxy compound neither needs to be monitored, nor is the same isolated. This shortcut reaction, to the applicant's knowledge, has not yet been used to establish a recyclable, industrially advantageous oxidation process.

Furthermore, it is beneficial that the mixture (solution/dispersion) generated in step (b) contains the nitrosonium ion essentially as sole oxidant. Therefore, the contact between a primary oxidant, for instance those strong oxidants which are typically used along with organic nitroxy compounds as catalyst in prior art processes, and the hydroxy compound substrate can be avoided. This minimizes undesired side reactions.

Step (b) is not specifically limited with respect to the concentration of the hydroxylamine in the aqueous or organic mixture and the reaction temperature. The hydroxylamine compound is preferably used in amounts being still soluble or dispersible in the mixture. Concentrations of 0.1 to 30 wt%, in particular 1-10 wt%, based on the aqueous or organic mixture are preferred. The reaction is preferably conducted at temperatures from 0-50°C, more preferably 10-30°C.

In line with **step (c)** of the claimed process, the mixture obtained in step (b), which contains the nitrosonium ion, is reacted with a second mixture containing the hydroxy compound to be oxidized. The nitrosonium ion can be added to this second mixture or vice versa. Following step (b), the mixture used in step (c) will contain the nitrosonium ion at least as major oxidant (in terms of molar amount), and preferably as sole oxidant. Thus, it is preferred that the nitrosonium ion is present in molar amounts of at least 80%, preferably at least 90%, more preferably at least 95%, based on the total molar amount of all oxidants. Other oxidants may still be present as impurities resulting from the preceding steps. As mentioned, their presence is, however, not preferred.

According to **step (d)**, the substrate hydroxy compound is oxidized by the nitrosonium ion, thereby generating the corresponding hydroxylamine. Depending on the pH of this last oxidation step, the hydroxylamine will be protonated or not.

The resulting hydroxylamine easily synproportionates with equimolar amounts of nitrosonium ion under weakly acidic, neutral or alkaline conditions.

It should be understood that step (d) is not restricted to the complete conversion of hydroxy compounds to the corresponding aldehyde and/or carboxy compound. Especially with high-molecular weight hydroxy compounds such as polysaccharides (e.g. pulp) it is often strongly preferred to conduct a partial oxidation only.

According to a preferred embodiment of the claimed process, this optionally protonated hydroxylamine is then recycled to step (a) where it is protonated, if necessary. This embodiment results in a closed cycle for the actual oxidative species, the nitrosonium ion. Furthermore, the nitroxy compound is at each stage of the claimed process fully converted to an optionally protonated hydroxylamine and/or nitrosonium ion. Since these are polar substances, in contrast to the nitroxy compound itself, the volatility of the oxidant species and resulting material losses are considerably reduced. Thereby, the overall efficiency of the process is increased.

The last step (d) of the claimed process can be conducted under conditions known in the art. Since the oxidant mixture generated in step (b) contains the nitrosium ion essentially as the sole oxidant, it can be applied in a wide pH range of preferably 1 to 11.5. For mechanistical reasons, as known in the art, oxidation reactions carried out at lower pH value tend to proceed slower. Preferably, the oxidation is carried out in a liquid medium such as the already mentioned aqueous or organic solvent. Furthermore, the following conditions are preferred.
- a reaction temperature of 0 to 50°C,
- a concentration of the hydroxy compound in the aqueous or organic solvent from 0.1 to 50 wt%, in particular 5 to 20 wt%, based on the weight of the solvent,
- a molar ratio hydroxy compound/nitrosonium ion of 0.1 to 5.

A particularly preferred concentration range for polysaccharides is 0.5 to 10 wt%.

The invention is now further illustrated by the following examples.

### Example 1

440 mg (2.8mmol) TEMPO were dissolved in 10 ml of water. To this mixture, 240 mg ascorbic acid (1.36 mmol) were added in order to reduce TEMPO to the corresponding hydroxylamine. An immediate reaction took place, which could be monitored by the decrease in color after a few minutes. The mixture was allowed to react for 60 minutes and became orange-brown. After lowering the pH to approximately 4 by the addition of diluted HCl, the color disappeared. Subsequently, 200 µl of a sodium hypochlorite solution (1.8M) in water were added. This resulted in an immediate increase and change of the color. This was repeated four times. After addition of altogether 1ml 1.8M-sodium hypochlorite solution, no further change was observed. By means of UV-vis spectroscopy it was confirmed that the hydroxylamine was completely converted to the nitrosonium ion.

This experiment proves that it is possible to convert the hydroxylamine directly into the corresponding nitrosonium ion under suitable process conditions. This nitrosonium ion can be used in the oxidation of hydroxy compounds under conditions known in the art.

### Example 2

1.10 g TEMPO (7.05mmol) was dissolved in 18 ml 0.5M HCl. Upon heating for 10 minutes at 40°C the material completely dissolved and an orange-brownish solution was obtained. The material was cooled to room temperature and added to neutralized hypochlorous acid/hypochlorite solution (2.5 mmol at pH 7.5) During the reaction the pH was kept between 4 and 7. A UV-Vis spectrum of the diluted solution showed a λₘₐₓ value at about 460 to 480nm, which is characteristic for nitrosonium compounds.

The mixture was added to a solution of 15g pulp (Grapho Celeste) in 1.51 water. The pH was adjusted to 6.3. An immediate decrease in pH was observed, due to reaction of the nitrosonium ion and cellulose. To maintain the pH between 6.5 and 7.5, sodium hydroxide had to be added (in total 8.5 mmol NaOH). After the reaction, the pulp was washed repeatedly with water to remove TEMPO and salt. In the modified pulp the aldehyde content was determined by titration with hydroxylamine (see Methods in Carbohydrate Chemistry, vol. III, page 49-50, AP, New York and London (1963)). The content amounted to 180 µmol/g. Taking into account the formation of some carboxylic acid groups, the content is an agreement with the theoretical amount.

### Example 3

640 mg of TEMPO was suspended in 10 ml 0.25 M hydrochloric acid. A solution of hypochlorous acid was prepared by diluting 1.5 ml 1.8 M hypochlorite to 10 ml and adjusting the diluted solution with 10% acetic acid solution to pH 4.5. To this acidified solution the TEMPO-solution was added. A gradual decrease in pH occurred. To maintain the pH between 3.5 and 5.8 ml 0.5M NaOH was added. The final pH was 4.5. The solution thus obtained was added to a suspension of 5 g pulp in 11 water. The nitrosonium ion was allowed to react with the pulp. An immediate drop of pH occurred. To keep the pH between 6 and 7, 2.0 ml NaOH (0.5M) had to be added during the reaction. After 2 hours, no further pH drop was observed, which indicated that the reaction was completed. The pulp was filtered off and repeatedly washed with water, until the filtrate was colorless. The amount of aldehyde groups formed was 150 µmol/g.

### Example 4

1g 4-acetamido TEMPO was suspended in 20 ml 0.2 M HCl. From the UV-vis spectrum it was seen that a disproportionation occurred. To the acidic solution a diluted non-neutralised sodium hypochlorite solution was added. During the reaction the pH was kept below 4. To avoid too high pH values, about 2ml 1M hydrochloric acid was added. Then the solution was reacted with a pulp suspension having a consistency of 10g/l under the same conditions as in example 3. The reaction visibly proceeded within 30 minutes while the color of the solution (brownish-yellow) turned into light yellow. The amount of aldehydes introduced in the pulp was 180 µmol/g.

### Example 5

To a disproportionated solution of 370 mg of TEMPO (pH 0.5) a solution of 1 ml 1.8 M NaOCl was added. During the pH gradually increased. At the end of the reaction the pH was 1.5. Then the brownish solution was brought to pH 6.9. 150 mg methyl α-D-glucopyransosie was added.An immediate decline in pH was observed. To keep the pH at 7, sodium hydroxide solution (1M) had to be added. After the reaction was finished, the uronic acid content was determined according to the uronic acid assay (Blumenkrantz and Asboe-Hansen, Anal Biochem. 54 (1973) 434). The uronic acid content in the reaction mixture was found to be 90 mg.

## Claims

1. A process for the oxidation of a hydroxy compound comprising the steps of:
a) providing a first mixture containing an organic protonated hydroxylamine compound,
b) oxidizing said protonated hydroxylamine compound to the corresponding nitrosonium compound,
c) reacting the nitrosonium compound obtained thereby with a second mixture containing a hydroxy compound, and
d) oxidizing said hydroxy compound by means of the nitrosonium compound, thereby generating the hydroxylamine.

2. A process according to claim 1 wherein the hydroxy compound comprises a primary hydroxy group.

3. A process according to claim 2 wherein the hydroxy compound is selected from oligo- or polysaccharides, preferably starch or cellulose.

4. A process according to claim 3 wherein the cellulose is present in cellulosic pulp and the oxidation is carried out in the absence of chlorine-containing oxidants.

5. A process according to any of claims 1 to 4 wherein the first mixture is an aqueous or organic solution or dispersion.

6. A process according to claim 1 wherein the protonated hydroxylamin compound is generated from an organic nitroxy compound by reduction or disproportionation.

7. A process according to claim 6 wherein said nitroxy compound has the following formula (V) 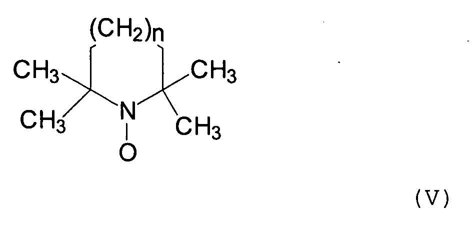 where n = 0 or 1 and where the methylene groups of the ring may carry one or more substituents selected from alkyl, alkoxy, aryl, aryloxy, amino, amido, oxo, cyano, hydroxy, carboxyl, phosphonooxy, maleimido, isothiocyanato, alkyloxy, fluorophosphinyloxy, substituted or unsubstituted benzoyloxy.

8. A process according to claim 6 or 7 wherein the nitroxy compound stems from a reaction mixture obtained by oxidizing a hydroxy compound in the presence of this nitroxy compound wherein the nitroxy compound may not have been fully reacted.

9. Process according to claim 1 wherein the oxidation of said protonated hydroxylamine compound to a nitrosonium ion compound is performed in the presence of hypochlorous acid or peracid.

10. Process according to claim 1 wherein the pH value of step (b) is 7 or less, in particular 6 or less.

11. Process according to any of the preceding claims wherein the hydroxylamine compound generated in step (d) is recycled to step (a).
